# EUROPÄISCHE PATENTSCHRIFT

(11) **EP 1 242 627 B1**
(45) Veröffentlichungstag und Bekanntmachung des Hinweises auf die Patenterteilung: **17.09.2003**
(21) Anmeldenummer: 00990530.8
(22) Anmeldetag: 13.12.2000
(51) Int. Cl.: C12Q 1/68, G01N 27/26, H01L 51/30

(54) **VERFAHREN UND VORRICHTUNG ZUM NACHWEIS UND ZUR QUANTIFIZIERUNG VON BIOMOLEKÜLEN**
METHOD AND DEVICE FOR DETECTING AND QUANTIFYING BIOMOLECULES
PROCEDE ET DISPOSITIF DE DETECTION ET DE QUANTIFICATION DE BIOMOLECULES

(30) Priorität: 13.12.1999 DE 19960076
(43) Veröffentlichungstag der Anmeldung: 25.09.2002
(73) Patentinhaber: november Aktiengesellschaft Gesellschaft für Molekulare Medizin, 91056 Erlangen (DE)
(72) Erfinder: SCHÜLEIN, Jürgen, 91054 Erlangen (DE); HASSMANN, Jörg, 91052 Erlangen (DE)
(74) Vertreter: Gassner, Wolfgang, Dr.
(86) Internationale Anmeldenummer: DE0004438
(87) Internationale Veröffentlichungsnummer: WO01044501

(56) Entgegenhaltungen:
- WO-A-00/60125
- WO-A-98/20162
- WO-A-98/31839
- US-A- 5 770 369
- H-W FINK ET AL: "Electrical conduction through DNA molecules" NATURE, MACMILLAN JOURNALS LTD. LONDON, GB, Bd. 398, 1. April 1999 (1999-04-01), Seiten 407-410, XP002162319 ISSN: 0028-0836

## Beschreibung

Die Erfindung betrifft ein Verfahren und eine Vorrichtung zum Nachweis und zur Quantifizierung eines in einer Lösung enthaltenen ersten Biomoleküls.

Nach dem Stand der Technik ist es bekannt, Polynukleotidsequenzen, wie DNA, durch voltammetrische Verfahren nachzuweisen. Dazu wird in der US 5,312,572 vorgeschlagen, redoxaktive Moleküle der die DNA enthaltenden Lösung zuzusetzen. Die redoxaktiven Moleküle binden im Falle der Hybridisierung der DNA an das gebildete doppelsträngige DNA-Molekül. Das redoxaktive Molekül bewirkt ein meßbares Redoxsignal.

Ein ähnliches Verfahren ist aus der US 5,871,918 bekannt. Aus der US 5,874,046 ist ein Sensorsystem bekannt, bei dem an einer Elektrode eine dotierte Oligonukliotidsequenz gebunden ist. Es wird die im Falle der Hybridisierung der Oligonukliotidsequenz auftretende erhöhte Leitfähigkeit z.B. mittels zyklischer Voltammetrie indirekt gemessen. Das Bereitstellen dotierter Oligonukleotide ist aufwendig. Die indirekte Messung der Leitfähigkeit mittels zyklischer Voltammetrie weist eine geringe Sensitivität auf.

Aus der WO 96/01836 ist ein sogenannter Chip zum Nachweis von Polynukleotidsequenzen bekannt. Der Chip ist aus einem z.B. Silizium hergestellten Substrat gebildet. Auf dem Chip sind eine Vielzahl miniaturisierter Reaktionsfelder vorgesehen. In jedem der Reaktionsfelder ist eine besondere Polynukleotidsequenz gebunden. Beim Eintauchen des Chips in eine die nachzuweisende Polynukleotidsequenz enthaltende Lösung kommt es zur Hybridisierung mit einer der besonderen Polynukleotidsequenzen. Die Hybridisierung wird mittels Fluoreszenz nachgewiesen.

Auch die WO 95/12808 betrifft ein Verfahren zum Nachweis von Polynukleotidsequenzen mittels Chip. Dabei wird über den Chip nach Art einer Elektrode eine Spannung angelegt. Geladene in der Lösung befindliche Polynukleotidsequenzen werden dadurch an der Oberfläche des Chips bzw. den dort vorgesehenen miniaturisierten Reaktionsfeldern angereichert.

Aus der US 5,591,578 ist eine voltammetrische Detektionsmethode zur Identifikation von einzelsträngigen Target-Nukleinsäuresequenzen bekannt. Dabei wird eine zur Target-Nukleinsäuresequenz komplementäre Nukleinsäuresequenz kovalent an die Elektrode gebunden. An dieser Nukleinsäuresequenz sind kovalent redoxaktive Übergangsmetallkomplexe gebunden. Im Falle der Hybridisierung ist ein erhöhtes Redoxsignal meßbar.

Die US 5,783,063 offenbart ein Verfahren zur Abschätzung von Parametern zur Charakterisierung von Nukleinsäure-Proben.

Die DE 198 08 884 beschreibt ein Verfahren zum Nachweis von chemischen Substanzen unter Verwendung von zwei in Wechselwirkung stehenden fluorophoren Gruppen, welche an ein Molekül gebunden sind. Bei spezifischer Anlagerung wird eine Wechselwirkung zwischen den fluorophoren Gruppen geändert.

In der DE 198 11 732 ist eine Mikrotiterplatte beschrieben. Diese ist mit kovalent gebundenen Oligonukleotiden beschichtet. Die Mikrotiterplatte kann zum Nachweis mittels PCR hergestellter Produkte verwendet werden.

Die WO 99/47700 bezieht sich auf ein Verfahren zum Nachweis von einer Nukleotidsequenz mittels Fluoreszenz. Dabei wird ein Molekül mit einer fluorophoren Gruppe an einer festen Phase gebunden. Bei Vorliegen der Targetsequenz wird eine zweite fluorophore Gruppe derart angebunden, daß ein strahlungsloser oder direkter Energieübertrag zwischen den fluorophoren Gruppen stattfinden kann.

Aus der WO 99/47701 ist ein Verfahren zum Nachweis einer Nukleotidsequenz mittels PCR bekannt. Bei der Durchführung der PCR werden drei Primer verwendet, wobei der dritte Primer an einer festen Phase gebunden ist. Er dient der Anreicherung des Reaktionsprodukts und der Verkürzung der Reaktionszeit.

Aus Kelly, S.O., Ang. Chem. Int. Ed 38 (7), 941 (1999), "Long-Range Elektron Transfer through DNA Films" ist es bekannt, daß in doppelsträngiger DNA ein Elektronentransfer mit einer langen Reichweite stattfinden kann.

WO 98/20162 offenbart die Verwendung von Nukleinsäuren mit elektronenübertragenden Teilen als Sonden. Beide elektronenübertragenden Teile sind an der ersten Sonde befestigt. Sobald diese Sonden mit einer eine Zielsequenz enthaltenden Lösung in Kontakt gebracht werden, kommt es zu einer Hybridisierung dieser Zielsequenz mit der dazu komplementären Sonde. Das gebildete doppelsträngige DNA-Molekül weist eine signifikant höhere elektrische Leitfähigkeit auf als die einzelsträngige Sonde.

US 5,770,369 offenbart ähnliche Verfahren zum Nachweis von Nukleinsäuren. Hierbei werden Nukleinsäuren kovalent mit einem Elektronendonor und einem Elektronenakzeptor verbunden, wonach anschließend die elektrische Leitfähigkeit gemessen wird. Die elektronenübertragenden Teile sind an der ersten Sonde oder an zwei Separaten Sonden befestigt die alleine oder zusammen mit einer Zielsequenz hybridisieren.

WO 98/31839 offenbart Verfahren zum Nachweis von Bindungen zwischen verschiedenen Biomoleküle. Diese Biomoleküle werden an verschiedene Elektroden angebracht.

Fink und Schönenberger, Nature V 398, 407 (1999), " Electrical conduction through DNA molecules" offenbaren die Leitfähigkeit von doppelsträngigen DNA-Molekülen.

Die nach dem Stand der Technik bekannten Verfahren zum Nachweis von DNA sind aufwendig. Sie erfordern z.B. den Zusatz redoxaktiver Moleküle. Eine exakte Quantifizierung der nachzuweisenden DNA bzw. Biomoleküle ist mit den bekannten Verfahren nicht möglich. Die zum Nachweis eingesetzte optische Detektion erfordert ferner einen hohen apparativen Aufwand.

Aufgabe der Erfindung ist es, die Nachteile nach dem Stand der Technik zu beseitigen. Es sollen insbesondere ein möglichst sensitives Verfahren sowie eine Vorrichtung für den gleichzeitigen Nachweis und die Quantifizierung geringer Mengen von in einer Lösung befindlichen Biomolekülen angegeben werden.

Diese Aufgabe wird durch die Merkmale der Ansprüche 1 und 21 gelöst. Zweckmäßige Ausgestaltungen ergeben sich aus den Merkmalen der Ansprüche 2 bis 20 und 22 bis 36.

Nach Maßgabe der Erfindung ist ein Verfahren zum Nachweis und/oder zur Quantifizierung eines in einer Lösung enthaltenen ersten Biomoleküls vorgesehen, mit folgenden Schritten:
a) Binden des ersten Biomoleküls an ein zweites Biomolekül, welches zum ersten Biomolekül zumindest abschnittsweise eine spezifische Affinität aufweist und
b) Messen der elektrischen Leitfähigkeit des aus dem ersten und dem zweiten Biomolekül gebildeten Komplexes, wobei das zweite Biomolekül eine Brücke zwischen einer ersten und einer zweiten Elektrode bildet.

Das vorgeschlagene Verfahren ist besonders sensitiv. Im Falle der Bildung eines z.B. aus erstem und zweitem Biomolekül gebildeten doppelsträngigen Komplexes ist es möglich, durch die zwischen der ersten und zweiten Elektrode gebildete Brücke eine Änderung der elektrischen Eigenschaften zu erfassen. Es sind also einzelne Biomoleküle in der Lösung detektierbar. Das ermöglicht auch die Quantifizierung kleinster Mengen.

Unter dem Begriff Biomolekül werden Moleküle verstanden, welche zumindest abschnittsweise ein spezifische Affinität zueinander aufweisen und bei Bildung einer gegenseitigen Bindung entlang des gebundenen Abschnitts veränderte elektrische Eigenschaften zeigen. Unter einem "Biomolekül" wird insbesondere ein aus Nukleinsäuren gebildetes Molekül verstanden. Ein solches Molekül weist zumindest zwei kovalent gebundene Nukleotide auf. Die Nukleinsäure enthält üblicherweise Phosphodiesther-Bindungen. Sie kann aber auch zum Beispiel Phosphoramid-, Phosphorthionat-, Phosphordithionat-, O-Methylphosphoroamideit-Bindungen oder Petitnukleinsäure-Bindungen aufweisen. Die Nukleinsäuren können einzel- oder doppelsträngig sein. Es kann sich um DNA, RNA oder ein Hybrid handeln, bei dem die Nukleinsäure eine Kombination von Desoxiribo- und Ribonukleinsäuren enthält. Auch Proteine, PNA und dgl. werden unter der Begriff "Biomolekül" verstanden.

Im Sinne der vorliegenden Erfindung weisen zwei Moleküle dann zueinander eine "spezifische" Affinität auf, wenn sie aufgrund attraktiver Wechselwirkungen, z.B. Wasserstoffbrückenbindungen, van-der-Waals-Kräfte ionischer oder kovalenter Bindung, eine Bindung eingehen können oder zumindest eine Tendenz besitzen, sich über schwache Wechselwirkungen, wie z.B. Wasserstoffbrückenbindungen oder dgl., gegenseitig zu binden. Eine solche spezifische Affinität besitzen z.B. Moleküle wie Antigen/Antikörper, Streptavidin/Biotin, Ligand/Rezeptor und komplementäre Nukleinsäuresequenzen.

Unter dem Begriff "Komplex" wird eine Assoziation aus mindestens zwei Biomolekülen verstanden, welche in Folge ihrer Affinität zueinander ein Konstrukt bilden.

Nach einer Ausgestaltung wird das zweite Biomolekül vor dem Schritt lit. a zumindest mit einem Ende an eine der Elektroden gebunden. Die Bindung des mindestens einen Endes des zweiten Biomoleküls an die eine Elektrode kann über eine direkte Bindung ein Spacer- und/oder ein Linker-Molekül vermittelt werden. Nach einer weiteren Ausgestaltung wird das zweite Biomolekül vor oder nach dem Schritt lit. a mit dem anderen Ende an die zweite Elektrode gebunden. Im letzteren Fall kann das Binden des anderen Endes durch Anlegen eines Potentials unterstützt werden. An das freie Ende des zweiten Biomoleküls kann ein im geeigneten Potentialbereich geladenes Molekül, im folgenden Ladungsträger genannt, gebunden sein. Das ermöglicht es insbesondere bei ungeladenen zweiten Biomolekülen, eine Brücke durch elektrostatische Kräfte zu bilden. Selbstverständlich können auch geladene zweite Biomoleküle mit einem Ladungsträger versehen sein. Bei dem Ladungsträger kann es sich z.B. um einen Metallcluster, ein organisches Molekül oder einen Komplexbildner handeln.

Es ist vorteilhaft, wenn der Schritt lit. a zwischen dem 3inden des ersten Endes des zweiten Biomoleküls an die erste Elektrode und dem Binden des zweiten Endes des zweiten Biomoleküls an die zweite Elektrode erfolgt. Beim Schritt lit. a kann die Bindung des ersten Biomoleküls an das zweite Biomolekül durch ein elektrisches Feld beschleunigt werden. Das elektrische Feld kann z.B. durch Anlegen eines Potentials an die Elektroden erzeugt werden. Vorteilhafterweise läßt sich durch das vorgeschlagene Verfahren die Stringenz auch durch Potentialänderungen variieren.

Die Wahl der jeweiligen Ausgestaltungsvarianten richtet sich nach der Art des ersten Biomoleküls. Dabei spielen die räumliche Ausdehnung des ersten Biomoleküls sowie dessen Ladung eine besondere Rolle.

Die erste und die zweite Elektrode sind zweckmäßigerweise auf einem elektrisch isolierenden Substrat aufgebracht. Nach einer weiteren Verfahrensvariante kann das Substrat vor dem Schritt lit. b gespült und/oder getrocknet und/oder evakuiert werden.

Nach einem weiteren Ausgestaltungsmerkmal wird die elektrische Leitfähigkeit des zwischen der ersten und der zweiten Elektrode gebundenen Komplexes gemessen. Anstatt der Leitfähigkeit kann auch die Kapazität und/oder die Impedanz des zwischen der ersten und der zweiten Elektrode gebundenen Komplexes gemessen werden. Die Messung der vorgenannten elektrischen Größen ermöglicht den Nachweis und/oder die Quantifizierung eines Biomoleküls, welches zumindest abschnittsweise eine Affinität zum zweiten Biomolekül aufweist.

Es hat sich als vorteilhaft erwiesen, daß der Abstand zwischen der ersten und der zweiten Elektrode 3 nm bis 1 µm, vorzugsweise 50nm, ist.

Das erste Biomolekül kann eine zum zweiten Biomolekül komplementäre einzelsträngige DNA oder RNA sein. Ferner kann das zweite Biomolekül auch abschnittsweise doppelsträngig ausgebildet sein, wobei der/die doppelsträngige/n Abschnitt/e vorzugsweise aus DNA und/oder RNA gebildet ist/sind.

In das zweite Biomolekül kann aber auch ein einzelsträngiger Abschnitt eingeschaltet sein; der einzelsträngige Abschnitt kann aus DNA, RNA oder PNA gebildet sein. Ferner kann in das zweite Biomolekül ein Protein oder ein Peptid eingeschaltet sein. Das ermöglicht beispielsweise den Nachweis von Antikörpern, sofern diese als erstes Biomolekül in der Lösung enthalten sind.

Die Sensitivität des Nachweises erster Biomoleküle läßt sich durch die jeweiligen Stringenzbedingungen einstellen. Die Stringenzbedingungen können beispielsweise durch eine Änderung der an den Elektroden angelegten Spannung geändert und an die jeweiligen Verhältnisse angepaßt werden.

Um eine Mehrfachdurchführung des Verfahrens zu gewährleisten, kann zum Entfernen des ersten Biomoleküls vom zweiten Biomolekül nach dem Schritt lit. b eine Kraft auf das erste Biomolekül mittels einer angelegten Spannung ausgeübt werden. Ferner kann das Substrat nach dem Schritt lit. b gespült und/oder geheizt werden. Durch das Heizen kann eine thermische Denaturierung der ersten Biomoleküle erreicht werden. Bestimmte erste Biomoleküle binden vorzugsweise bei einer bestimmten vorgegebenen Temperatur. Durch Heizen bzw. Einstellen der Temperatur kann die Spezifität des Verfahrens weiter erhöht werden. Die Spezifität kann auch durch eine Variation des pH-Werts der Lösung erhöht oder durch eine bestimmte Salzkonzentration eingestellt werden.

Zur Verbesserung der Leitfähigkeit des aus dem ersten und dem zweiten Biomolekül gebildeten Komplexes können der Lösung Interkalatoren zugesetzt sein. Dabei handelt es sich um Moleküle, welche sich an bzw. in doppelsträngige Moleküle an- bzw. einlagern und Hoppingzentren bilden oder über andere Mechanismen eine erhöhte Leitfähigkeit zur Folge haben.

Nach weiterer Maßgabe der Erfindung ist eine Vorrichtung zum Nachweis und/oder zur Quantifizierung eines in einer Lösung enthaltenen ersten Biomoleküls vorgesehen, wobei
aa) eine erste und eine zweite Elektrode auf einem elektrisch isolierenden Substrat aufgebracht sind,
bb) zumindest an der ersten Elektrode mit seinem einen Ende ein zweites Biomolekül gebunden ist, welches zum ersten Biomolekül zumindest abschnittsweise eine spezifische Affinität aufweist und wobei
cc) der Abstand zwischen der ersten und der zweiten Elektrode so gewählt ist, daß durch Binden des anderen Endes des zweiten Biomoleküls eine Brücke zwischen der ersten und der zweiten Elektrode herstellbar ist.
Die erfindungsgemäße Vorrichtung ermöglicht einen besonders sensitiven Nachweis sowie eine Quantifizierung von in der Lösung vorliegenden ersten Biomolekülen.

Die Elektroden können aus elektrisch leitfähigen Materialien wie z.B. Gold, einem leitfähigen Kunststoff oder Graphit bestehen. Des weiteren können die Elektroden auch in Form von Mikroelektroden, z.B. Nanotubes, ausgebildet sein.

Nach einer Ausgestaltung ist/sind die Elektrode/n mit im Bereich der gewählten Spannungen redoxinaktiven Molekülen beschichtet. Eine solche Beschichtung trägt zur Unterdrückung von verunreinigungsbedingten Leitfähigkeitsphänomenen bei.

Hinsichtlich weiterer vorteilhafter Ausgestaltungen der Vorrichtung wird auf die vorangegangene Beschreibung verwiesen.

Nach einer weiteren vorrichtungsseitigen Ausgestaltung sind eine Vielzahl erster und zweiter Elektroden auf dem Substrat angebracht. Vorteilhafterweise sind die an die ersten Elektroden gebundenen zweiten Biomoleküle voneinander verschieden, so daß ein gleichzeitiger Nachweis und/oder eine Quantifizierung einer Vielzahl erster Biomoleküle möglich ist. Der erfindungsgemäße Chip hat den Vorteil, daß damit auf einfache Weise ein Nachweis und/oder eine Quantifizierung in einer Lösung enthaltener erster Biomoleküle möglich ist. Die Quantitfizierung kann digital erfolgen. Dabei kann man sich vorteilhafterweise den Umstand zu Nutze machen, daß jede Brücke ein besonderes Signal liefert. Es kann insbesondere auf die einen hohen apparativen Aufwand erfordernden optischen Detektionseinrichtungen verzichtet werden.

Nachfolgend werden anhand der Zeichnung Ausführungsbeispiele der Erfindung näher erläutert. Hierin zeigen:
- Fig. 1: ein erstes Ausführungsbeispiel einer erfindungsgemäßen Vorrichtung,
- Fig. 2: ein zweites Ausführungsbeispiel einer erfindungsgemäßen Vorrichtung,
- Fig. 3: ein drittes Ausführungsbeispiel einer erfindungsgemäßen Vorrichtung,
- Fig. 4: schematisch eine erste Verfahrensvariante,
- Fig. 5: schematisch eine zweite Verfahrensvariante,
- Fig. 6: schematisch eine dritte Verfahrensvariante,
- Fig. 7a - c: schematisch die Teilschritte einer vierten Verfahrensvariante und
- Fig. 8a - d: schematisch die Teilschritte und das Meßergebnis einer fünften Verfahrensvariante.

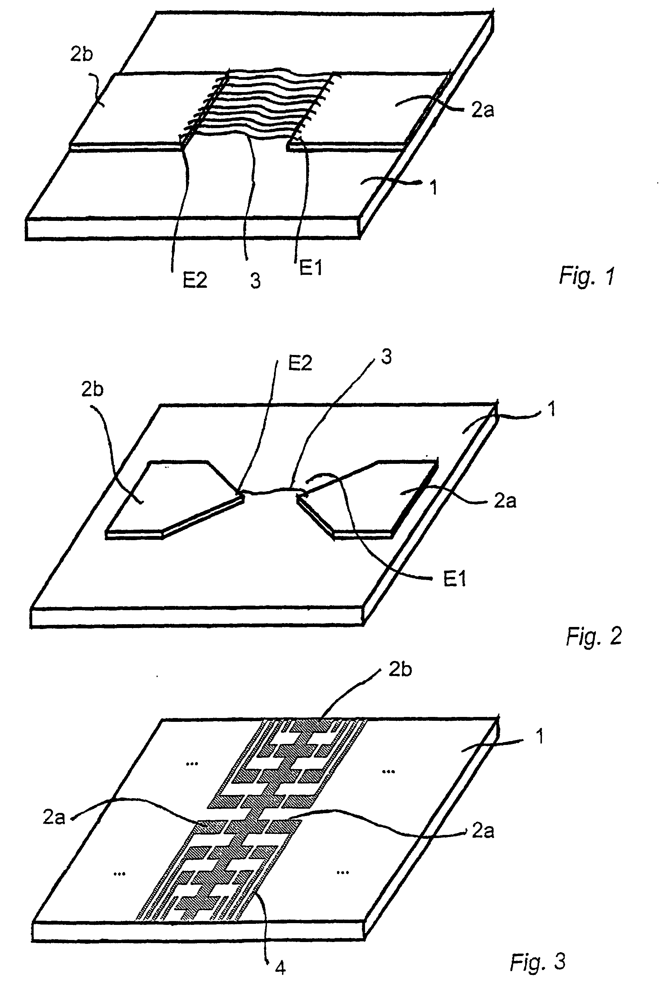

In den Fig. 1 bis 3 ist mit 1 ein z.B. aus Silizium, Silizium mit Oxidschicht, Quarzkristall, Glas oder Glimmer hergestelltes im wesentlichen elektrisch isolierendes Substrat bezeichnet. Auf dem Substrat 1 aufgebracht sind eine erste 2a und eine zweite Elektrode 2b. In den Fig. 1 und 2 sind die elektrischen Verbindungen der Elektroden 2a, 2b mit einer (hier nicht dargestellten) Meßeinrichtung aus Gründen der Klarheit nicht gesondert dargestellt. Eine einzelsträngige DNA 3 ist mit ihrem einen Ende E1 an die erste Elektrode 2a und mit ihrem anderen Ende E2 an die zweite Elektrode 2b gebunden. Sie bildet also eine brückenartige Verbindung zwischen den beiden Elektroden 2a und 2b.

Es ist auch möglich, daß z.B. nur das eine Ende E1 an die ersten Elektrode 2a gebunden ist, während das zweite Ende E2 zunächst frei beweglich ist. An das zweite Ende E2 ist in diesem Fall zweckmäßigerweise ein Dentrimer gebunden. Das zweite Ende E2 kann durch Anlegen eines geeigneten Potentials an die zweite Elektrode 2b angezogen und mittels des Goldclusters gebunden werden.

In Fig. 3 ist ein erfindungsgemäßer Chip gezeigt. Dabei liegt eine Vielzahl diskreter erster Elektroden 2a einer gemeinsamen zweiten Elektrode bei 2b gegenüber. Jede der ersten Elektroden 2a ist mit einer separaten Zuleitung 4 versehen. Der Chip ist nach herkömmlicher lithographischer Technik hergestellt. Die die erste 2a und die zweite Elektrode 2b verbindenden Biomoleküle sind hier aus Gründen der Übersichtlichkeit nicht dargestellt.

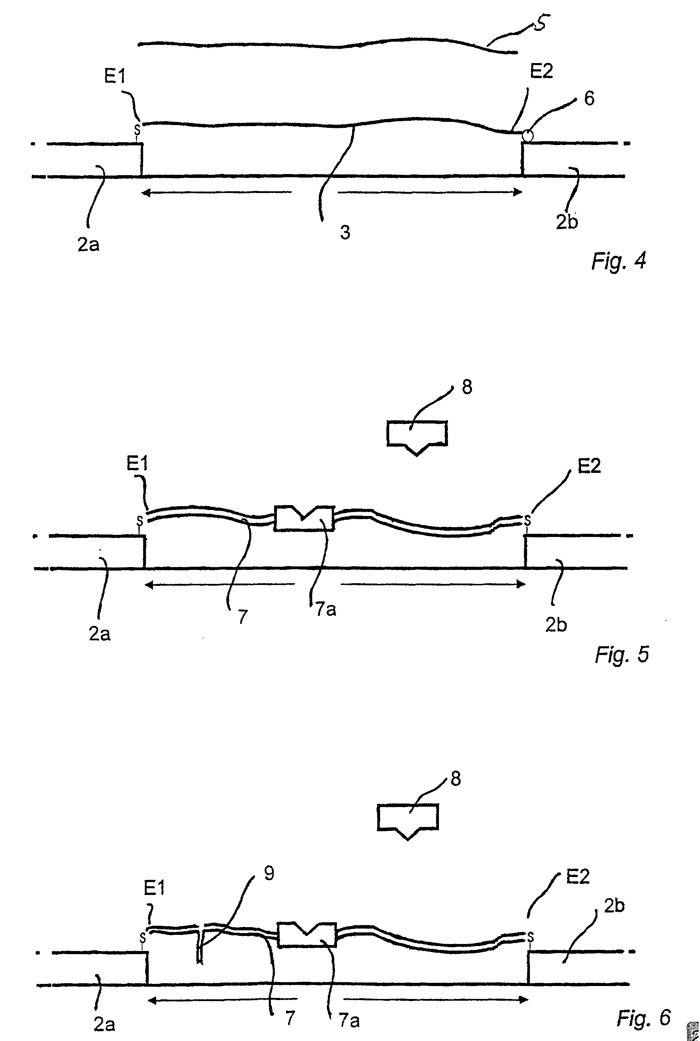

In der Fig. 4 ist schematisch eine erste Variante zum Nachweis eines einzelsträngigen ersten DNA-Moleküls 5 gezeigt. An die erste Elektrode 2a ist über ein mit S bezeichnetes Spacer-Molekül das zweite einzelsträngige DNA-Molekül 3 gebunden. Am anderen Ende E2 des zweiten DNA-Moleküls 3 ist ein Goldcluster 6 vorgesehen. Durch Anlegen eines Potentials ist hier zunächst eine brückenartige Verbindung mittels des zweiten DNA-Moleküls 3 zwischen der ersten 2a und der zweiten Elektrode 2b hergestellt worden. Der Goldcluster 6 beschleunigt die Bindung des anderen Endes E2 mit der zweiten Elektrode 2b. Das in der Lösung befindliche erste DNA-Molekül 5 ist zum zweiten DNA-Molekül 3 komplementär. Sobald die gezeigte Vorrichtung mit der Lösung in Kontakt gebracht wird, kommt es zu einer Hybridisierung des zweiten DNA-Moleküls 3 mit dem dazu komplementärem ersten DNA-Molekül 5. Das gebildete doppelsträngige DNA-Molekül weist eine signifikant höhere elektrische Leitfähigkeit auf, als das die erste 2a und die zweite Elektrode 2b verbindende einzelsträngige zweite DNA-Molekül. Diese besondere erhöhte elektrische Leitfähigkeit tritt insbesondere nur dann auf, wenn eine weitgehend vollständige Hybridisierung vorliegt. Mittels des beschriebenen Verfahrens kann mit einer hohen Sensitivität das Vorliegen erster DNA-Moleküle 5 in der Lösung detektiert werden.

Die in Fig. 5 gezeigte zweite Verfahrensvariante eignet sich insbesondere zum Nachweis von Antikörpern 8. Die Verbindung zwischen der ersten 2a und der zweiten Elektrode 2b ist hier durch ein abschnittsweise doppelsträngiges Biomolekül 7 ausgebildet, dessen Enden E1 und E2 jeweils mittels eines Spacer-Moleküls S an die erste 2a bzw. die zweite Elektrode 2b gebunden sind. In das zweite Biomolekül 7 ist eine einzelsträngige Peptidsequenz 7a eingeschaltet. Bei Vorliegen eines dazu korrespondierenden Antikörpers in der Lösung bindet dieser an die einzelsträngige Peptidsequenz 7a. Die Leitfähigkeit des gebildeten Konstrukts ist erhöht. Damit gelingt auf einfache Weise ein Nachweis von in der Lösung befindlichen Antikörpern 8.

Bei der in Fig. 6 gezeigten dritten Verfahrensvariante ist in einem Strang des Biomoleküls 7 eine Haarnadelschleife 9 ausgebildet. Im Bereich der Haarnadelschleife 9 ist der gegenüberliegende Strang unterbrochen. Bei der Bindung des Antikörpers 8 an den dazu korrespondierenden einzelsträngigen Peptidabschnitt 7a kommt es zu einer Konformationsänderung. Das gebildete Konstrukt zieht sich zusammen. Die Haarnadelschleife 9 wird gestreckt. Die die Unterbrechung begrenzenden Enden des gegenüberliegenden Strangs entfernen sich voneinander. Eine zuvor beobachtbare Leitfähigkeit nimmt bei der Bindung des Antikörpers 8 an den einzelsträngigen Peptidabschnitt 7a ab. Damit ist ein einfacher Nachweis von in der Lösung befindlichen Antikörpern 8 möglich.

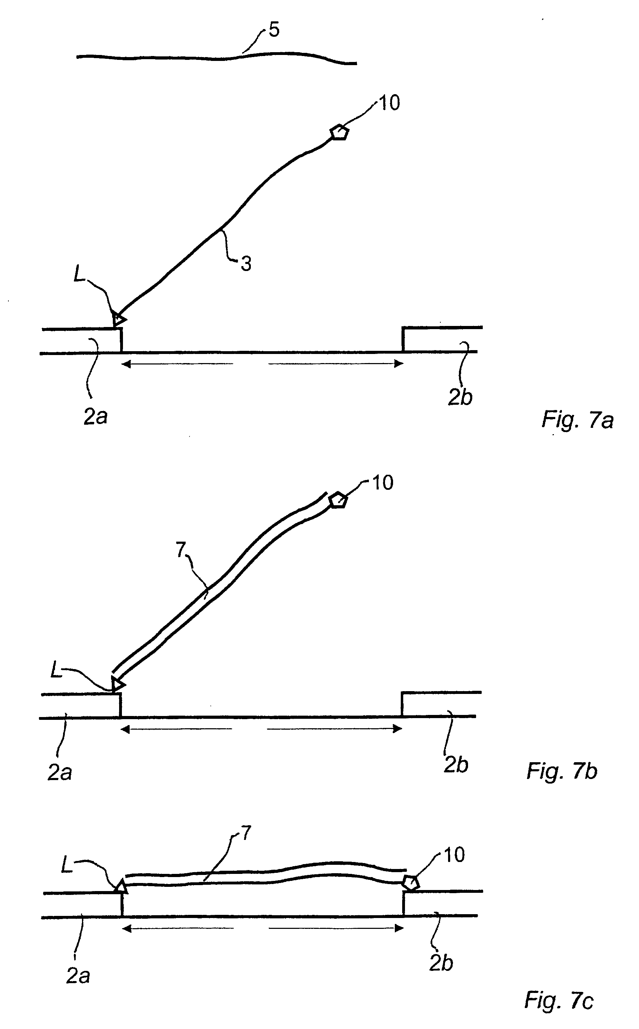

In Fig. 7a bis 7c ist eine vierte Verfahrensvariante gezeigt. Eine zu einem ersten Biomolekül, z.B. einem ersten DNA-Molekül 5, komplementäres zweites Biomolekül, z.B. ein zweites DNA-Molekül 3, ist über einen Linkers L an die erste Elektrode 2a gebunden. Am anderen Ende des zweiten DNA-Moleküls 3 ist ein geladenes Molekül 10 gebunden. Beim Inkontakbringen mit einer das erste DNA-Molekül 5 enthaltenden Lösung hybridisiert das erste DNA-Molekül 5 mit dem zweiten DNA-Molekül 3. Danach wird gespült. Anschließend wird eine Spannung zwischen der ersten 2a und der zweiten Elektrode 2b angelegt. Auf das geladene Molekül 10 wird eine Kraft ausgeübt. Das geladene Molekül 10 wird an die zweite Elektrode 2b angezogen (siehe Fig. 7c). Es bildet sich eine Brücke. Über die gebildete Brücke kann die Leitfähigkeit des doppelsträngigen Komplexes unmittelbar mittels einer einfachen Strom-Spannungsmessungen ermittelt werden. Die Messung kann auch bei Abwesenheit der Lösung, d.h. im trockenen Zustand erfolgen.

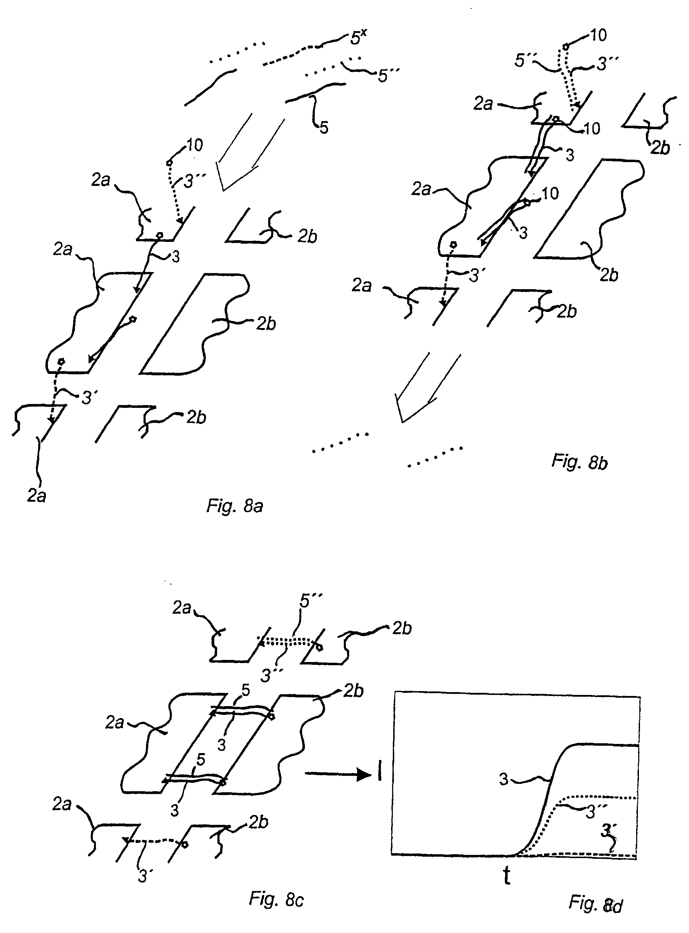

Die Fig. 8a bis 8c zeigen eine fünfte Verfahrensvariante; Fig. 8d zeigt schematisch ein dabei beobachtetes Meßergebnis. In den Fig. 8a bis 8c ist ein Abschnitt eines Arrays gezeigt, welches mehrere erste 2a und zweite Elektroden 2b enthält. An die ersten Elektroden 2a sind unterschiedliche zweite Biomoleküle, z.B. zweite DNA-Moleküle 3, 3'und 3'', mittels eines Linkers gebunden. Beim Inkontaktbringen eines solchen Elektroden-Arrays mit einer unterschiedliche erste Biomoleküle, z.B. erste DNA-Moleküle 5, 5^{x} und 5'' enthaltenden Lösung kommt es je nach der Affinität der ersten DNA-Moleküle 5, 5^{x} und 5'' zu den zweiten DNA-Molekülen 3, 3' und 3'' zu einer Hybridisierung. Im vorliegenden Beispiel hybridisiert das erste DNA-Molekül 5 mit dem zweiten DNA-Molekül 3 und das erste DNA-Molekül 5'' mit dem zweiten DNA-Molekül 3''. Das zweite DNA-Molekül 3' ist nicht komplementär zum der Lösung enthaltenen ersten DNA-Molekül 5^{x}. Nach dem Spülen des Elektroden-Arrays wird zwischen der ersten 2a und der zweiten Elektrode 2b eine Spannung angelegt, so daß durch die Biomoleküle eine Brücke zwischen den beiden Elektroden 2a, 2b gebildet wird. Es wird nun die Leitfähigkeit zwischen jedem Elektrodenpaar 2a, 2b gemessen. Das dabei erzielte Meßergebnis ist schematisch in Fig. 8d wiedergegeben. Die höchste Leitfähigkeit zeigt das Elektrodenpaar, welches zwei Brücken mit hybridisierten DNA-Molekülen 3, 5 aufweist. Die Leitfähigkeit bei der Bildung lediglich einer Brücke ist etwa halb so groß. Sie ist bei dem aus dem DNA-Molekülen 3'' und 5'' beobachtbar. Die allein durch das zweite DNA-Molekül 3' gebildete Brücke führt dagegen kaum zu einer unmittelbaren Leitfähigkeit zwischen den Elektroden 2a, 2b.

Mit dem vorgeschlagenen Verfahren können hoch sensitiv in einer Lösung enthaltene erste Biomoleküle detektiert werden. Die Sensitivität kann insbesondere auch durch die Belegungsdichte der ersten Elektrode 2a erhöht werden.

### Bezugszeichenliste

- 1: Substrat
- 2a, 2b: erste, zweite Elektrode
- 3, 3',3'': zweite DNA-Moleküle
- 4: Zuleitung
- 5, 5', 5'': erste DNA-Moleküle
- 6: Goldcluster
- 7: Biomolekül
- 7a: einzelsträngiger Peptidabschnitt
- 8: Antikörper
- 9: Haarnadelschleife
- 10: geladenes Molekül

- E1: Ende
- E2: zweites Ende
- S: Spacer
- L: Linker

## Patentansprüche

1. Verfahren zum Nachweis und/oder zur Quantifizierung eines in einer Lösung enthaltenen ersten Biomoleküls (5, 8), mit folgenden Schritten:
a) Binden des ersten Biomoleküls (5, 8) an ein zweites Biomolekül (3, 7), welches zum ersten Biomolekül (5, 8) zumindest abschnittsweise eine spezifische Affinität aufweist, so dass das erste (5,8) und das zweite Biomolekül (3,7) einen Komplex bilden, und
b) Bildung einer Brücke zwischen einer ersten (2a) und einer zweiten Elektrode (2b) mittels des zweiten Biomoleküls (3, 7), und
c) Messen der elektrischen Leitfähigkeit des aus dem ersten (5,8) und dem zweiten Biomolekül (3,7) gebildeten Komplexes unmittelbar mittels einer Strom-/Spannungmessung.

2. Verfahren nach Anspruch 1, wobei das zweite Biomolekül (3,7) vor dem Schritt lit. a zumindest mit einem Ende (E1) an eine der Elektroden (2a, 2b) gebunden wird.

3. Verfahren nach einem der vorhergehenden Ansprüche, wobei die Bindung mindestens eines Endes (E1) des zweiten Biomoleküls (3,7) an die eine Elektrode (2a, 2b) über einen Spacer-(S) und/oder ein Linker-Molekül vermittelt wird.

4. Verfahren nach einem der vorhergehenden Ansprüche, wobei das zweite Biomolekül (3,7) vor oder nach dem Schritt lit. a mit dem anderen Ende (E2) an die zweite Elektrode (2b) gebunden wird.

5. Verfahren nach Anspruch 3, wobei das Binden des anderen Endes (E2) durch Anlegen eines Potentials unterstützt wird.

6. Verfahren nach einem der vorhergehenden Ansprüche, wobei an das andere Ende (E2) des zweiten Biomoleküls (3, 7) ein Ladungsträger (6) gebunden ist.

7. Verfahren nach Anspruch 6, wobei der Ladungsträger (6) ein Metallcluster, ein organisches Molekül oder ein Komplexbildner ist, der die Bindung des anderen Endes (E2) an die zweite Elektrode (2b) über den Ladungsträger (6) vermittelt.

8. Verfahren nach einem der vorhergehenden Ansprüche, wobei der Schritt lit. a zwischen den Binden des einen Endes (E1) des zweiten Biomoleküls (3, 7) an die erste Elektrode (2a) und dem Binden des anderen Endes (E2) des zweiten Biomoleküls (3, 7) an die zweite Elektrode (2b) erfolgt.

9. Verfahren nach einem der vorhergehenden Ansprüche, wobei die erste (2a) und die zweite Elektrode (2b) auf einem elektrisch isolierenden Substrat (1) aufgebracht sind.

10. Verfahren nach einem der vorhergehenden Ansprüche, wobei das Substrat (1) vor dem Schritt lit. b gespült und/oder getrocknet und/oder evakuiert wird.

11. Verfahren nach einem der vorhergehenden Ansprüche, wobei die elektrische Leitfähigkeit des zwischen der ersten (2a) und der zweiten Elektrode (2b) gebundenen Komplexes gemessen wird.

12. Verfahren nach einem der vorhergehenden Ansprüche, wobei anstatt der Leitfähigkeit die Kapazität des zwischen der ersten (2a) und der zweiten Elektrode (2b) gebundenen Komplexes gemessen wird.

13. Verfahren nach einem der vorhergehenden Ansprüche, wobei anstatt der Leitfähigkeit die Impedanz des zwischen der ersten (2a) und der zweiten Elektrode (2b) gebundenen Konstrukts gemessen wird.

14. Verfahren nach einem der vorhergehenden Ansprüche, wobei der Abstand zwischen der ersten (2a) und der zweiten Elektrode (2b) 3 nm bis 1 µm, vorzugsweise 50 nm, ist.

15. Verfahren nach einem der vorhergehenden Ansprüche, wobei das erste Biomolekül (5, 8) eine zum zweiten Biomolekül (3,7) komplementäre einzelsträngige DNA oder RNA ist.

16. Verfahren nach einem der vorhergehenden Ansprüche, wobei das zweite Biomolekül (3,7) abschnittsweise doppelsträngig ausgebildet ist, wobei der/die doppelsträngige/n Abschnitt/e vorzugsweise aus DNA und/oder RNA gebildet ist/sind.

17. Verfahren nach einem der vorhergehenden Ansprüche, wobei in das zweite Biomolekül (3,7) ein einzelsträngiger Abschnitt eingeschaltet ist.

18. Verfahren nach einem der vorhergehenden Ansprüche, wobei der einzelsträngige Abschnitt aus DNA, RNA oder PNA gebildet ist.

19. Verfahren nach einem der vorhergehenden Ansprüche, wobei das zweite Biomolekül (3,7) mit einem Protein oder einem Peptid assoziiert ist.

20. Verfahren nach einem der vorhergehenden Ansprüche, wobei zum Entfernen des ersten Biomoleküls (5, 8) vom zweiten Biomolekül (3, 7) nach dem Schritt lit. b eine Kraft auf das erste Biomolekül (5, 8) mittels einer angelegten Spannung ausgeübt wird.

21. Verfahren nach einem der vorhergehenden Ansprüche, wobei zum Entfernen des ersten Biomoleküls (5, 8) vom zweiten Biomolekül (3, 7) das Substrat (1) nach dem Schritt lit. b gespült wird.

22. Vorrichtung zum Nachweis und/oder zur Quantifizierung eines in einer Lösung enthaltenen ersten Biomoleküls (5, 8), wobei
aa) eine erste (2a) und eine zweite Elektrode (2b) auf einem elektrisch isolierenden Substrat (1) aufgebracht sind,
bb) zumindest an der ersten Elektrode (2a) mit seinem einen Ende (E1) ein zweites Biomolekül (3, 7) gebunden ist, welches zum ersten Biomolekül (5, 8) zumindest abschnittsweise eine spezifische Affinität aufweist, und wobei
cc) der Abstand zwischen der ersten (2a) und der zweiten Elektrode (2b) so gewählt ist, daß durch Binden des anderen Endes (E2) des zweiten Biomoleküls (3, 7) eine Brücke zwischen der ersten (2a) und der zweiten Elektrode (2b) herstellbar ist.

23. Vorrichtung nach Anspruch 22, wobei der Abstand zwischen der ersten (2a) und der zweiten Elektrode (2b) 3 nm bis 1 µm, vorzugsweise 50 nm, ist.

24. Vorrichtung nach Anspruch 22 oder 23, wobei zumindest das eine Ende (E1) des zweiten Biomoleküls (3,7) an die eine Elektrode (2a, 2b) über eine direkte Kopplung, einen Spacer-(S) und/oder ein Linker-Molekül (L) gebunden ist.

25. Vorrichtung nach einem der Ansprüche 22 bis 24, wobei an das andere Ende (E2) des zweiten Biomoleküls (3, 7) ein Ladungsträger (6) gebunden ist.

26. Vorrichtung nach einem der Ansprüche 22 bis 25, wobei der Ladungsträger (6) ein Metallcluster, ein organisches Molekül oder ein Komplexbildner ist und die Bindung des anderen Endes (E2) an die zweite Elektrode (2b) über den Ladungsträger (6) vermittelbar ist.

27. Vorrichtung nach einem der Ansprüche 22 bis 26, wobei das Substrat (1) aus Keramik, aus Siliziumverbindungen, vorzugsweise Silizium mit Oxidschicht, aus Glimmer oder aus einer elektrisch isolierenden Polymermatrix hergestellt ist.

28. Vorrichtung nach einem der Ansprüche 22 bis 27, wobei eine mit der ersten (2a) und der zweiten Elektrode (2b) verbundene Einrichtung zum Messen der elektrischen Leitfähigkeit des aus dem ersten (5,8) und dem zweiten Biomolekül (3,7) gebildeten Konstrukts vorgesehen ist.

29. Vorrichtung nach Anspruch 28, wobei mittels der Einrichtung anstatt der Leitfähigkeit die Kapazität des aus dem ersten (5, 8) und dem zweiten Biomolekül (3, 7) gebildeten Konstrukts meßbar ist.

30. Vorrichtung nach Anspruch 28, wobei mittels der Einrichtung anstatt der Leitfähigkeit die Impedanz des aus dem ersten (5, 8) und dem zweiten Biomolekül (3, 7) gebildeten Konstrukts meßbar ist.

31. Vorrichtung nach einem der Ansprüche 22 bis 30, wobei das erste Biomolekül (5, 8) eine zum zweiten Biomolekül (3, 7) komplementäre einzelsträngige DNA oder RNA ist.

32. Vorrichtung nach Anspruch 31, wobei das zweite Biomolekül (3, 7) abschnittsweise doppelsträngig ausgebildet ist, wobei die doppelsträngigen Abschnitte vorzugsweise aus DNA und/oder RNA gebildet ist/sind.

33. Vorrichtung nach Anspruch 32, wobei in das zweite Biomolekül (3, 7) ein einzelsträngiger Abschnitt eingeschaltet ist.

34. Vorrichtung nach einem der Ansprüche 22 bis 33, wobei der einzelsträngige Abschnitt aus DNA, RNA oder PNA gebildet ist.

35. Vorrichtung nach einem der Ansprüche 22 bis 34, wobei in das zweite Biomolekül (3, 7) ein Protein oder ein Peptid eingeschaltet ist.

36. Vorrichtung nach einem der Ansprüche 22 bis 35, wobei eine Vielzahl erster (2a) und zweiter Elektroden (2b) auf dem Substrat (1) angebracht sind.

37. Vorrichtung nach einem der Ansprüche 22 bis 36, wobei die an die ersten Elektroden (2a) gebundenen zweiten Biomoleküle (3, 7) voneinander verschieden sind, so daß ein gleichzeitiger Nachweis und/oder Quantifizierung einer Vielzahl erster Biomoleküle (5, 8) möglich ist.

## Claims

1. A method for detecting and/or quantifying a first biomolecule (5, 8) contained in a solution, having the following steps:
a) binding the first biomolecule (5, 8) to a second biomolecule (3, 7), which at least in some sections has a specific affinity to the first biomolecule so that the first (5, 8) and the second biomolecule (3, 7) form a complex, and
b) forming a bridge between a first (2a) and a second electrode (2b) by means of the second biomolecule (3, 7), and
c) measuring the electric conductivity of the complex made from the first (5, 8) and the second biomolecule (3, 7) directly by means of a current/voltage measurement.

2. A method according to Claim 1, whereby the second biomolecule (3, 7) is bound to one of the electrodes (2a, 2b) at least by one end (E1) before step a).

3. A method according to one of the preceding Claims, whereby the binding of at least one end (E1) of the second biomolecule (3, 7) to the one electrode (2a, 2b) is produced via a spacer (S) and/or a linker molecule.

4. A method according to one of the preceding Claims, whereby the second biomolecule (3, 7) is bound by the other end (E2) to the second electrode (2b) before or after step a).

5. A method according to Claim 3, whereby the binding of the other end (E2) is supported by applying a potential.

6. A method according to one of the preceding Claims, whereby a charge carrier (6) is bound to the other end (E2) of the second biomolecule (3, 7).

7. A method according to Claim 6, whereby the charge carrier (6) is a metal cluster, an organic molecule or a complexing agent which produces the binding of the other end (E2) to the second electrode (2b) via the charge carrier (6).

8. A method according to one of the preceding Claims, whereby step a) takes place between the binding of the one end (E1) of the second biomolecule (3, 7) to the first electrode (2a) and the binding of the other end (E2) of the second biomolecule (3, 7) to the second electrode (2b).

9. A method according to one of the precharacterising Claims, whereby the first (2a) and the second electrode (2b) are applied to an electrically insulating substrate (1).

10. A method according to one of the preceding Claims, whereby the substrate (1) is rinsed and/or dried and/or evacuated prior to step b).

11. A method according to one of the preceding Claims, whereby the electrical conductivity of the complex bound between the first (2a) and the second electrode (2b) is measured.

12. A method according to one of the preceding Claims, whereby, instead of the conductivity, the capacitance of the complex bound between the first (2a) and the second electrode (2b) is measured.

13. A method according to one of the preceding Claims, whereby, instead of the conductivity, the impedance of the construct bound between the first (2a) and the second electrode (2b) is measured.

14. A method according to one of the preceding Claims, whereby the distance between the first (2a) and the second electrode (2b) is 3 nm to 1 µm, preferably 50 nm.

15. A method according to one of the preceding Claims, whereby the first biomolecule (5, 8) is a single-stranded DNA or RNA complementary to the second biomolecule (3, 7).

16. A method according to one of the preceding Claims, whereby the second biomolecule (3, 7) has a double-stranded structure in some sections, with the double-stranded section(s) preferably being made from DNA and/or RNA.

17. A method according to one of the preceding Claims, whereby a single-stranded section is incorporated into the second biomolecule (3, 7).

18. A method according to one of the preceding Claims, whereby the single-stranded section is made from DNA, RNA or PNA.

19. A method according to one of the preceding Claims, whereby the second biomolecule (3, 7) is associated with a protein or a peptide.

20. A method according to one of the preceding Claims, whereby a force is exerted on the first biomolecule (5, 8) by means of an applied voltage to remove the first biomolecule (5, 8) from the second biomolecule (3, 7) after step b).

21. A method according to one of the preceding Claims, whereby the substrate (1) is rinsed after step b) to remove the first biomolecule (5, 8) from the second biomolecule (3, 7).

22. A device for detecting and/or quantifying a first biomolecule (5, 8) contained in a solution, wherein
aa) a first (2a) and a second electrode (2b) are applied to an electrically insulating substrate (1),
bb) a second biomolecule (3, 7), which has a specific affinity to at least some sections of the first biomolecule (5, 8), is bound at least to the first electrode (2a) with its one end (E1), and wherein
cc) the distance between the first (2a) and the second electrode (2b) is chosen so that by binding the other end (E2) of the second biomolecule (3, 7) a bridge can be produced between the first (2a) and the second electrode (2b).

23. A device according to Claim 22, wherein the distance between the first (2a) and the second electrode (2b) is 3 nm to 1 µm, preferably 50 nm.

24. A device according to Claim 22 or 23, wherein at least the one end (E1) of the second biomolecule (3, 7) is bound to the one electrode (2a, 2b) via a direct coupling, a spacer (S) and/or a linker molecule (L).

25. A device according to one of Claims 22 to 24, wherein a charge carrier (6) is bound to the other end (E2) of the second biomolecule (3, 7).

26. A device according to one of Claims 22 to 25, wherein the charge carrier (6) is a metal cluster, an organic molecule or a complexing agent and the binding of the other end (E2) to the second electrode (2b) can be produced via the charge carrier (6).

27. A device according to one of Claims 22 to 26, wherein the substrate (1) is produced from ceramics, from silicon compounds, preferably silicon having an oxide layer, from mica or from an electrically insulating polymer matrix.

28. A device according to one of Claims 22 to 27, wherein an instrument connected to the first (2a) and the second electrode (2b) is provided to measure the electric conductivity of the construct formed from the first (5, 8) and the second biomolecule (3, 7).

29. A device according to Claim 28, wherein, instead of the conductivity, the capacitance of the construct formed from the first (5, 8) and the second biomolecule (3, 7) can be measured by means of the instrument.

30. A device according to Claim 28, wherein, instead of the conductivity, the impedance of the construct formed from the first (5, 8) and the second biomolecule (3, 7) can be measured by means of the instrument.

31. A device according to one of Claims 22 to 30, wherein the first biomolecule (5, 8) is a single-stranded DNA or RNA complementary to the second biomolecule (3, 7).

32. A device according to Claim 31, wherein the second biomolecule (3, 7) has a double-stranded construction in some sections, with the double-stranded sections being preferably made from DNA and/or RNA.

33. A device according to Claim 32, wherein a single-stranded section is incorporated into the second biomolecule (3, 7).

34. A device according to one of Claims 22 to 33, wherein the single-stranded section is made from DNA, RNA or PNA.

35. A device according to one of Claims 22 to 34, wherein a protein or a peptide is incorporated into the second biomolecule (3, 7).

36. A device according to one of Claims 22 to 35, wherein a plurality of first (2a) and second electrodes (2b) are mounted on the substrate (1).

37. A device according to one of Claims 22 to 36, wherein the second biomolecules (3, 7) bound to the first electrodes (2a) are different from each other, so that it is possible simultaneously to detect and/or quantify a plurality of first biomolecules (5, 8).

## Revendications

1. Procédé de détection et/ou de quantification d'une première biomolécule (5, 8) contenue dans une solution, comportant les étapes suivantes :
a) liaison de la première biomolécule (5, 8) à une seconde biomolécule (3, 7) qui présente une affinité spécifique, au moins par segments, vis-à-vis de la première biomolécule (5, 8), de sorte que la première (5, 8) et la seconde (3, 7) biomolécules forment un complexe, et
b) formation d'un pont entre une première (2a) électrode et une seconde (2b) électrode au moyen de la seconde biomolécule (3, 7), et
c) mesure de la conductivité électrique du complexe formé à partir de la première (5, 8) et de la seconde (3, 7) biomolécules directement au moyen d'une mesure de courant/tension.

2. Procédé selon la revendication 1, dans lequel la seconde biomolécule (3, 7) est liée avant l'étape 1.a au moins par une extrémité (E1) à une des électrodes (2a, 2b).

3. Procédé selon l'une des revendications précédentes, dans lequel la liaison d'au moins une extrémité (E1) de la seconde biomolécule (3, 7) à une électrode (2a, 2b) est réalisée par l'intermédiaire d'une molécule d'espacement (S) et/ou d'une molécule de liaison.

4. Procédé selon l'une des revendications précédentes, dans lequel la seconde biomolécule (3, 7), avant ou après l'étape 1.a, est liée par l'autre extrémité (E2) à la seconde électrode (2b).

5. Procédé selon la revendication 3, dans lequel la liaison de l'autre extrémité (E2) est soutenue par application d'un potentiel.

6. Procédé selon l'une des revendications précédentes, dans lequel un porteur de charge (6) est lié à l'autre extrémité (E2) de la seconde biomolécule (3, 7).

7. Procédé selon la revendication 6, dans lequel le porteur de charge (6) est un agrégat métallique, une molécule organique ou un agent complexant, qui réalise la liaison de l'autre extrémité (E2) à la seconde électrode (2b), par l'intermédiaire du porteur de charge (6).

8. Procédé selon l'une des revendications précédentes, dans lequel l'étape 1.a se fait entre la liaison d'une extrémité (E1) de la seconde biomolécule (3, 7) à la première électrode (2a) et la liaison de l'autre extrémité (E2) de la seconde biomolécule (3, 7) à la seconde électrode (2b).

9. Procédé selon l'une des revendications précédentes, dans lequel la première (2a) et la seconde (2b) électrodes sont appliquées sur un substrat (1) électriquement isolant.

10. Procédé selon l'une des revendications précédentes, dans lequel le substrat (1) est lavé ou rincé et/ou séché et/ou évacué avant l'étape 1.b.

11. Procédé selon l'une des revendications précédentes, dans lequel on mesure la conductivité électrique du complexe lié entre la première (2a) et la seconde (2b) électrodes.

12. Procédé selon l'une des revendications précédentes, dans lequel, au lieu de la conductivité, on mesure la capacité du complexe lié entre la première (2a) et la seconde (2b) électrodes.

13. Procédé selon l'une des revendications précédentes, dans lequel, au lieu de la capacité, on mesure l'impédance du produit de recombinaison lié entre la première (2a) et la seconde (2b) électrodes.

14. Procédé selon l'une des revendications précédentes, dans lequel l'écart entre la première (2a) et la seconde (2b) électrodes est compris entre 3 nm et 1 µm, et est de préférence égal à 50 nm.

15. Procédé selon l'une des revendications précédentes, dans lequel la première biomolécule (5, 8) est un ADN ou un ARN simple brin complémentaire de la seconde biomolécule (3, 7).

16. Procédé selon l'une des revendications précédentes, dans lequel la seconde biomolécule (3, 7) est conçue en tant que double brin par segments, le ou les segment(s) double brin étant de préférence formé(s) à partir d'ADN et/ou d'ARN

17. Procédé selon l'une des revendications précédentes, dans lequel un segment simple brin est incorporé dans la seconde biomolécule (3, 7).

18. Procédé selon l'une des revendications précédentes, dans lequel le segment simple brin est formé à partir d'ADN, d'ARN ou d'ANP.

19. Procédé selon l'une des revendications précédentes, dans lequel une protéine ou un peptide est associé(e) à la seconde biomolécule (3, 7).

20. Procédé selon l'une des revendications précédentes, dans lequel, pour enlever la première biomolécule (5, 8) de la seconde biomolécule (3, 7) après l'étape 1.b, on exerce une force sur la première biomolécule (5, 8) au moyen d'une tension appliquée.

21. Procédé selon l'une des revendications précédentes, dans lequel, pour enlever la première biomolécule (5, 8) de la seconde biomolécule (3, 7), on rince ou lave le substrat (1) après l'étape 1.b.

22. Dispositif de détection et/ou de quantification d'une première biomolécule (5, 8) contenue dans une solution, dans lequel
aa) on applique une première (2a) et une seconde (2b) électrodes sur un substrat (1) électriquement isolant,
bb) au moins au niveau de la première électrode (2a), une seconde biomolécule (3, 7) est liée par une de ses extrémités (E1), laquelle biomolécule présente une affinité spécifique, au moins par segments, vis-à-vis de la première biomolécule (5, 8), et où
cc) l'écart entre la première (2a) et la seconde (2b) électrodes est sélectionné de façon telle que, par la liaison de l'autre extrémité (E2) de la seconde biomolécule (3, 7), on peut fabriquer un pont entre la première (2a) et la seconde (2b) électrodes.

23. Dispositif selon la revendication 22, dans lequel l'écart entre la première (2a) et la seconde (2b) électrodes est compris entre 3 nm et 1 µm, et est de préférence égal à 50 nm.

24. Dispositif selon la revendication 22 ou 23, dans lequel au moins une extrémité (E1) de la seconde biomolécule (3, 7) est liée à une électrode (2a, 2b) par un couplage direct, une molécule d'espacement (S) et/ou une molécule de liaison (L).

25. Dispositif selon l'une des revendications 22 à 24, dans lequel un porteur de charge (6) est lié à l'autre extrémité (E2) de la seconde biomolécule (3, 7).

26. Dispositif selon l'une des revendications 22 à 25, dans lequel le porteur de charge (6) est un agrégat métallique, une molécule organique ou un agent complexant, et la liaison de l'autre extrémité (E2) à la seconde électrode (2b) peut être réalisée par l'intermédiaire du porteur de charge (6).

27. Dispositif selon l'une des revendications 22 à 26, dans lequel le substrat (1) est fabriqué à partir de céramique, de composés de silicium, de préférence de silicium avec une couche d'oxyde, de mica ou d'une matrice polymère électriquement isolante.

28. Dispositif selon l'une des revendications 22 à 27, dans lequel est prévu un dispositif, relié à la première (2a) et à la seconde (2b) électrodes, de mesure de la capacité électrique du produit de recombinaison formé à partir de la première (5, 8) et de la seconde (3, 7) biomolécules.

29. Dispositif selon la revendication 28, dans lequel, au moyen du dispositif, on peut mesurer, au lieu de la conductivité, la capacité du produit de recombinaison formé à partir de la première (5, 8) et de la seconde (3, 7) biomolécules.

30. Dispositif selon la revendication 28, dans lequel, au moyen du dispositif, on peut mesurer, au lieu de la capacité, l'impédance du produit de recombinaison formé à partir de la première (5, 8) et de la seconde (3, 7) biomolécules.

31. Dispositif selon l'une des revendications 22 à 30, dans lequel la première biomolécule (5, 8) est un ADN ou un ARN simple brin complémentaire de la seconde biomolécule (3, 7).

32. Dispositif selon la revendication 31, dans lequel la seconde biomolécule (3, 7) est conçue en tant que double brin par segments, les segments double brin étant de préférence formés à partir d'ADN et/ou d'ARN.

33. Dispositif selon la revendication 32, dans lequel un segment simple brin est incorporé dans la seconde biomolécule (3, 7).

34. Dispositif selon l'une des revendications 22 à 33, dans lequel le segment simple brin est formé à partir d'ADN, d'ARN ou d'ANP.

35. Dispositif selon l'une des revendications 22 à 34, dans lequel une protéine ou un peptide est incorporé(e) dans la seconde biomolécule (3, 7).

36. Dispositif selon l'une des revendications 22 à 35, dans lequel on applique sur le substrat (1) un certain nombre de premières (2a) et de secondes (2b) électrodes.

37. Dispositif selon l'une des revendications 22 à 36, dans lequel les secondes biomolécules (3, 7) liées aux premières électrodes (2a) sont différentes les unes des autres, si bien qu'il est possible de détecter et/ou de quantifier simultanément plusieurs premières biomolécules (5, 8).
